# EUROPÄISCHE PATENTSCHRIFT

(11) **EP 1 306 065 B1**
(45) Veröffentlichungstag und Bekanntmachung des Hinweises auf die Patenterteilung: **30.04.2008**
(21) Anmeldenummer: 01126733.3
(22) Anmeldetag: 09.11.2001
(51) Int. Cl.: A61F 5/01

(54) **Quengel-Orthese**
Grumble orthesis
Orthese de geindre

(30) Priorität: 17.10.2001 DE 20117080 U
(43) Veröffentlichungstag der Anmeldung: 02.05.2003
(73) Patentinhaber: Jacobs, Klaus-Jürgen, 64546 Mörfelden-Walldorf (DE)
(72) Erfinder: Jacobs, Klaus-Jürgen, 64546 Mörfelden-Walldorf (DE)
(74) Vertreter: Ruckh, Rainer Gerhard

(56) Entgegenhaltungen:
- EP-A- 0 564 734
- EP-A- 1 138 286
- WO-A-00/09066
- US-A- 3 976 057
- US-A- 5 144 943
- US-A- 5 285 773
- US-A- 5 462 518
- US-A- 5 653 680
- US-A- 5 662 693

## Beschreibung

Die Erfindung betrifft eine Quengel-Orthese gemäß dem Oberbegriff des Anspruchs 1.

Derartige Quengel-Orthesen beziehungsweise Quengelschienen werden in den Bereichen der Chirurgie oder Orthopädie eingesetzt, um versteifte menschliche Gelenke oder Fehlstellungen von menschlichen Extremitäten zu korrigieren. Die Quengel-Orthese wird dabei gelenkübergreifend an den jeweiligen Extremitäten so angebracht, dass das in einer krankhaften Beuge- oder Streckstellung fixierte Gelenk in seiner Bewegungseinschränkung korrigiert wird. Die Quengel-Orthese weist hierzu typischerweise zwei gegeneinander schwenkbare Orthesenteile auf, die mittels eines mechanischen Quengelzuges bzw. Quengeldruckes zueinander bewegt werden, wodurch das Gelenk der in der Quengel-Orthese liegenden Extremität der jeweiligen Normalposition nähergebracht wird.

Die mechanischen Quengelzüge arbeiten mit an Hebeln der Quengel-Orthese angreifenden Zug- oder Druckfedern, elastischen Zügen, Zahnspindelgelenken, Seilzügen oder dergleichen.

Nachteilig bei derartigen Systemen ist, dass bei derartigen Systemen keine reproduzierbare und kontrollierbare Einstellung der Quengelkräfte, die auf das Gelenk wirken, möglich sind.

Insbesondere ist nachteilig, dass damit eine Kontrolle des Therapieerfolges während des Verlaufs der Gelenkbehandlung nicht möglich ist.

Besonders nachteilig hierbei ist, dass bei fortschreitender Korrektur des Gelenkes die Anpassung der Quengelkräfte nur ungenau möglich ist.

Aus der EP 0 564 734 A1 ist eine Quengelorthese bekannt, bei welcher zwei gelenkig verbundene Orthesenteile über flexible Transmissionselemente Quengelkräfte betätigbar sind. Durch Pneumatikelemente können Kräfte auf die Transmissionselemente ausgeübt werden.

Die US 5,144,943 beschreibt eine Vorrichtung zur Wiederherstellung der Beweglichkeit eines Fersengelenks eines Menschen. Die Vorrichtung umfasst eine an der Wade befestigbare Manschette und ein Fersenteil, in welches der Fuß des Patienten einsetzbar ist. Diese beiden Elemente sind allein über einen eine Feder, aufweisenden Zylinder verbunden. Mit der Federkraft wird eine Streckkraft zwischen den beiden Elementen bewirkt.

Die EP 1 138 286 A1 beschreibt eine Kraftunterstützungseinheit, die zur Verstärkung von Muskelkräften eines Menschen dient. Diese Einheit kann beispielsweise von zwei Manschetten gebildet sein, wobei eine Manschette die Hand einer Person und eine zweite Manschette den Unterarm der Person umschließt. Zwischen beiden Manschetten ist gelenkig ein Aktor, der vorzugsweise pneumatisch arbeitet, vorgesehen.

Der Erfindung liegt die Aufgabe zugrunde eine Quengel-Orthese der eingangs genannten Art so auszubilden, dass eine kontrollierte Therapie von Versteifungen und Fehlstellungen anatomischer Gelenke ermöglicht wird.

Zur Lösung dieser Aufgabe sind die Merkmale des Anspruchs 1 vorgesehen. Vorteilhafte Ausführungsformen und zweckmäßige Weiterbildungen der Erfindung sind in den Unteransprüchen beschrieben.

Die erfindungsgemäße Quengel-Orthese zur Korrektur von Gelenk-Fehlstellungen weist zwei gelenkig miteinander verbundene Orthesenteile mit einem Stellglied zur Erzeugung einer auf das Gelenk wirkenden Quengelkraft auf. Es ist eine pneumatische Korrektureinheit vorgesehen, welche als Grundkörper einen am ersten Orthesenteil befestigten Hohlzylinder und als Stellglied eine im Hohlzylinder verschiebbar gelagerte Kolbenstange aufweist. Das zweite Orthesenteil weist eine in Wirkverbindung mit dem Stellglied stehende Halterung auf, so dass bei Betätigung des Stellglieds die Orthesenteile gegeneinander schwenkbar sind. Die Verschiebung der Kolbenstange erfolgt mittels eines Faltenbalgs, der von einer Steuereinheit mit Druckluft versorgt wird.

Der Grundgedanke der Erfindung besteht somit darin, anstelle mechanischer Systeme ein über eine Steuereinheit pneumatisch gesteuertes Drucksteuersystem beziehungsweise Druckregelsystem einzusetzen, um die Quengelkräfte vorzugeben. Dabei wird über die Steuereinheit der Druck im Grundkörper exakt und reproduzierbar vorgegeben. Die durch den Druck bewirkte Auslenkung des Stellgliedes und die dadurch ausgeübte Quengelkraft der Quengel-Orthese ist damit ebenfalls exakt und reproduzierbar vorgebbar. Dabei erfolgt die Vorgabe der Quengelkräfte auch über längere Zeiträume genau entsprechend der vom Anwender wählbaren Vorgaben und kann zudem über entsprechende Anzeigen an der Steuereinheit kontrolliert werden. Dadurch ist eine vollständige und genaue Therapiekontrolle während der gesamten Behandlungsdauer gewährleistet.

Mit dem erfindungsgemäßen pneumatischen Druckregelsystem ist bei geringem konstruktiven Aufwand eine besonders genaue Vorgabe des Druckes im Hohlzylinder möglich und damit eine genaue Einstellung der Quengelkräfte. Es hat sich als zweckmäßig erwiesen, zur Erzeugung der Quengelkräfte mit geringem Luftdruck im Hohlzylinder zu arbeiten. Besonders vorteilhaft liegt der Luftdruck im Faltenbalg des Hohlzylinders im Bereich zwischen 533,3 Pa mol 333305 Pa (0.4 und 250 cm/Hg).

Dabei ist aus Gründen der Platzierbarkeit am jeweiligen Körper das Volumen im Innern des Faltenbalgs erheblich kleiner als das Volumen des Luftreservoirs in der Steuereinheit. Damit können bei der Behandlung auftretende schnelle Druckschwankungen im Faltenbalg, die durch plötzliche Gelenkbewegungen entstehen können, rasch ausgeglichen werden. Dies ist insbesondere bei einem Einsatz von Quengel-Orthesen zur Korrektur spastisch verursachter Kontraktionen der Fall. Bei einem einsetzenden Spasmus wird durch die plötzliche Gelenkbewegung ein Kraftstoß auf die Kolbenstange ausgeübt, so dass diese in den Hohlzylinder eingedrückt wird. Die dadurch bewirkte Druckerhöhung im Innern des Faltenbalgs wird durch die Ankopplung an das volumige Luftreservoir schnell ausgeglichen. Diese Eigenschaft der erfindungsgemäßen Quengel-

Orthese ist eine wesentliche Voraussetzung dafür, dass mit dieser Beugekontraktionen bei spastischen Patienten beeinflusst werden können.

Die Erfindung wird im nachstehenden anhand der Zeichnungen erläutert. Es zeigen:
- Figur 1:: Draufsicht auf die Handinnenseite einer als Handorthese ausgebildete Quengel-Orthese.
- Figur 2:: Seitenansicht der Handorthese gemäß Figur 1.
- Figur 3:: Perspektivische Darstellung einer als Sichelfußorthese ausgebildeten Quengel-Orthese.
- Figur 4:: Draufsicht auf die Unterseite der Sichelfußorthese gemäß Figur 3.
- Figur 5:: Pneumatische Korrektureinheit mit einer Befestigungs- und Justiereinheit für eine Quengel-Orthese gemäß den Figuren 1 und 2 in einem Längsschnitt sowie in einer Querschnittsdarstellung.
- Figur 6:: Detaildarstellungen von Komponenten der Befestigungs- und Justiereinheit gemäß Figur 5.
- Figur 7:: Blockschaltbild einer Steuereinheit für eine Quengel-Orthese gemäß den Figuren 1 - 4.

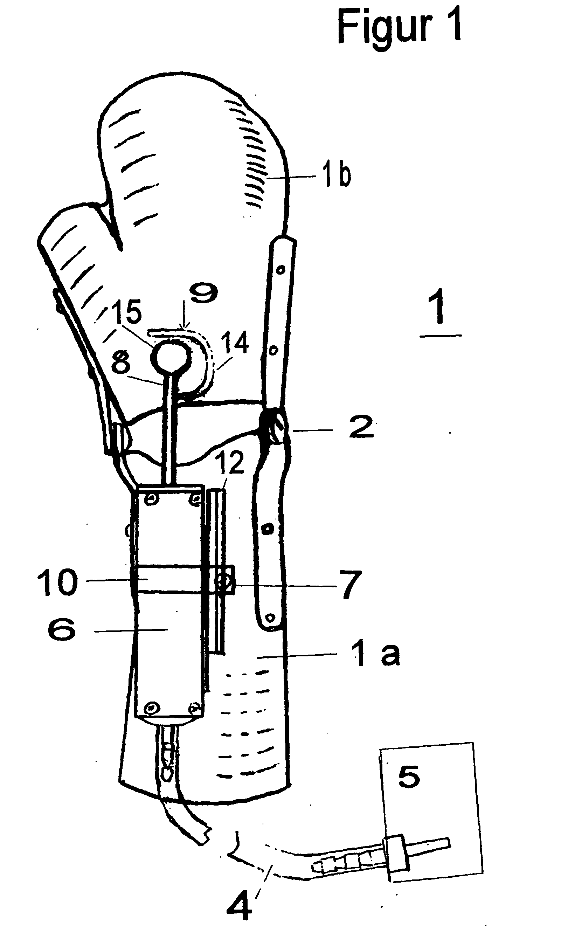

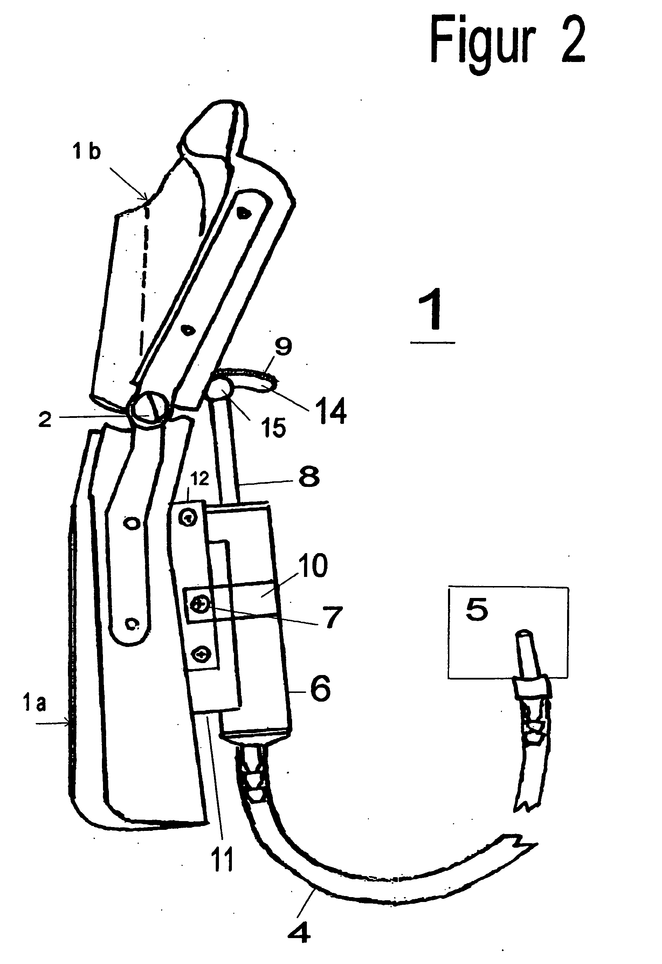

Die Figuren 1 und 2 zeigen ein erstes Ausführungsbeispiel der erfindungsgemäßen Quengel-Orthese 1. Die Quengel-Orthese 1 ist in diesem Fall als Handorthese ausgebildet und dient zur Therapie von Versteifungen oder Fehlstellungen menschlicher Handgelenke.

Die Handorthese besteht im Wesentlichen aus einem ersten Orthesenteil 1a und einem zweiten Orthesenteil 1b, die über eine Gelenkverbindung 2 gelenkig miteinander verbunden sind. Das erste Orthesenteil 1a dient zur Lagerung eines Unterarmes, das zweite Orthesenteil 1b dient zur Lagerung der anschließenden Hand.

An der Handorthese ist eine Korrektureinheit befestigt, die im vorliegenden Fall als pneumatische Korrektureinheit 3 ausgebildet ist. Prinzipiell können auch hydraulische Korrektureinheiten oder dergleichen vorgesehen sein. Die pneumatische Korrektureinheit 3 ist über einen Schlauch 4 an eine Steuereinheit 5 angeschlossen.

Die über die Steuereinheit 5 gesteuerte pneumatische Korrektureinheit 3 dient zur Vorgabe einer Quengelkraft, mit welcher das Handgelenk in eine vorgegebene Richtung gequengelt wird.

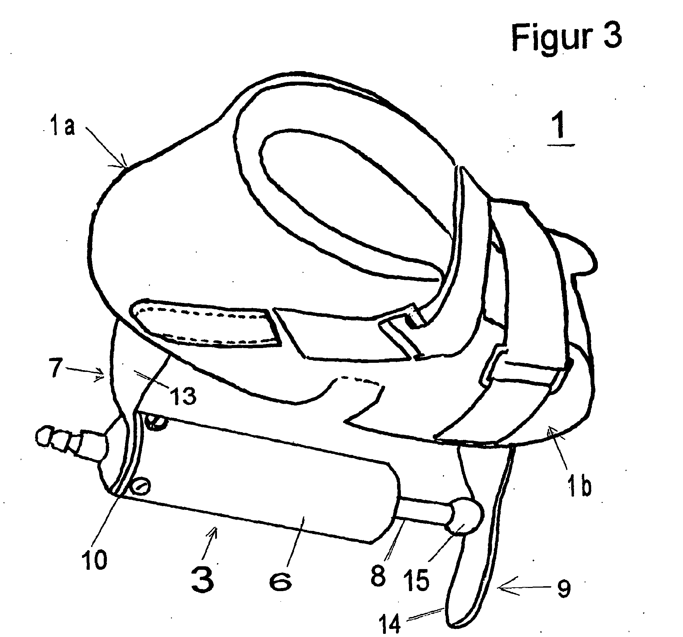

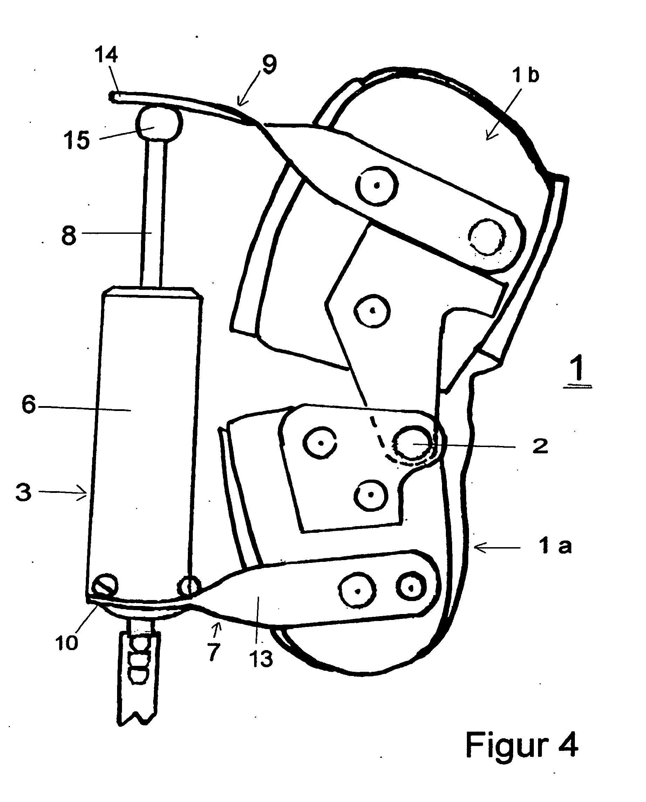

Die Figuren 3 und 4 zeigen ein zweites Ausführungsbeispiel der erfindungsgemäßen Quengel-Orthese 1. Die Quengel-Orthese 1 ist in diesem Fall als Sichelfußorthese ausgebildet und dient zur Korrektur von Fehlstellungen menschlicher Füße.

Analog zu dem Ausführungsbeispiel gemäß den Figuren 1 und 2 weist die Sichelfußorthese wiederum zwei Orthesenteile 1a, 1b auf, die über eine Gelenkverbindung 2 gelenkig miteinander verbunden sind. Die Vorgabe der Quengelkraft erfolgt wiederum über die pneumatische Korrektureinheit 3, die von der in den Figuren 3 und 4 nicht separat dargestellten Steuereinheit 5 angesteuert wird. Da in diesem Fall der Auslenkweg orthesentypisch kurz ist, kann auf eine entsprechende Justierung verzichtet werden.

Bei den in den Figuren 1 - 4 dargestellten Ausführungsbeispielen weist die pneumatische Korrektureinheit 3 als Grundkörper einen Hohlzylinder 6 auf, der jeweils mittels einer ersten Halterung 7 an dem ersten Orthesenteil 1a befestigt ist. Weiterhin weist die pneumatische Korrektureinheit 3 als Stellglied eine Kolbenstange 8 auf, die in Wirkverbindung mit einer zweiten Halterung 9 am zweiten Orthesenteil 1b steht.

Der hintere Teil der Kolbenstange 8 ist in dem Hohlzylinder 6 in dessen Längsrichtung verschiebbar gelagert. Durch eine über die Steuereinheit 5 gesteuerte Druckbeaufschlagung des Hohlzylinders 6 wird das Stellglied betätigt, das heißt die Kolbenstange 8 ausgelenkt. Durch die gelenkübergreifende Fixierung des Hohlzylinders 6 einerseits und der Kolbenstange 8 andererseits wird durch die Auslenkung der Kolbenstange 8 eine bestimmte Korrekturstellung der Quengel-Orthese 1 erreicht und damit eine Quengelkraft auf das in der Quengel-Orthese 1 gelagerte menschliche Gelenk ausgeübt.

Die Halterung 7 am ersten Orthesenteil 1a weist einen Haltering 10 auf, welche den Hohlzylinder 6 an seiner Mantelfläche umschließt. Bei dem Ausführungsbeispiel gemäß den Figuren 1 und 2 ist dieser Haltering 10 an einer Befestigungs- und Justiereinheit 11 montiert. Die Befestigungs- und Justiereinheit 11 ist mittels Befestigungsmittel 12, die im vorliegenden Fall von Schraubverbindungen gebildet sind, an dem ersten Orthesenteil 1a befestigt. Mittels der Befestigungs- und Justiereinheit 11 kann der Hohlzylinder 6 zu dessen Justierung in dessen Längsrichtung an dem ersten Orthesenteil 1a verschoben und dann in einer Sollposition mit dem Haltering 10 fixiert werden.

Bei dem Ausführungsbeispiel gemäß den Figuren 3 und 4 erfolgt die Fixierung des Hohlzylinders 6 mittels eines Haltearms 13. Der Haltearm 13 ist am ersten Orthesenteil 1a befestigt und steht von diesem seitlich ab.

Die Halterung 9 am zweiten Orthesenteil 1 b ist von einem zweiten Haltearm 14 gebildet. Der Haltearm 14 steht von dem zweiten Orthesenteil 1b derart ab, dass ein Segment dieses Haltearms 14 ein Widerlagerbildet, gegen welches das Vorderende der Kolbenstange 8 gedrückt wird. Wie aus den Figuren 1 - 4 ersichtlich weist die Kolbenstange 8 an ihrem vorderen Ende ein kugelförmiges Druckelement 15 auf, welches an dem Widerlager des Haltearms 14 anliegt.

Bei dem Ausführungsbeispiel gemäß den Figuren 1 und 2 ist der Haltearm 14 am zweiten Orthesenteil 1b bogenförmig ausgebildet, wobei das Vorderende des Haltearmes 14 das Widerlager bildet. Bei dem Ausführungsbeispiel gemäß den Figuren 3 und 4 verläuft der Haltearm 14 im Wesentlichen geradlinig. Auch in diesem Fall bildet das Vorderende des Haltearms 14 das Widerlager.

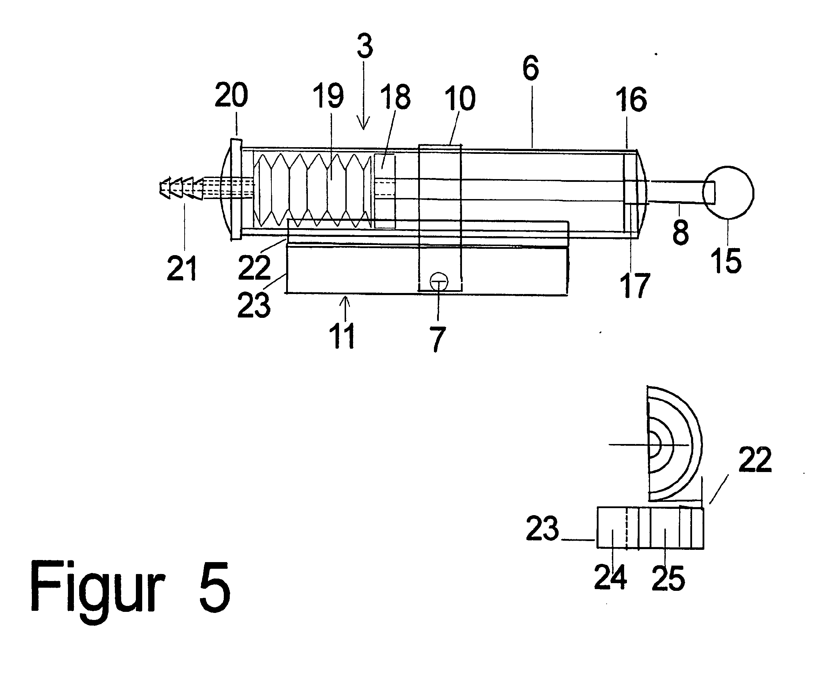

Der Aufbau der pneumatische Korrektureinheit 3 mit der Befestigungs- und Justiereinheit 11 gemäß dem Ausführungsbeispiel gemäß den Figuren 1 und 2 ist in Figur 5 detailliert dargestellt.

Wie aus Figur 5 ersichtlich ist das hintere Ende der Kolbenstange 8 in dem Hohlzylinder 6 der pneumatischen Korrektureinheit 3 geführt, während das vordere Ende mit dem kugelförmigen Druckelement 15 über das vordere Ende des Hohlzylinders 6 hervorsteht. Der Hohlzylinder 6 besteht vorzugsweise aus einem Aluminiumrohr oder aus kohlefaserverstärktem Kunststoff.

Die Kolbenstange 8 liegt in der Symmetrieachse des Hohlzylinders 6 und ist in Längsrichtung des Hohlzylinders 6 verschiebbar gelagert. Zur Lagerung der Kolbenstange 8 ist ein Verschlusskopf 16 vorgesehen, welcher am Hohlzylinder 6 an seiner vorderen Stirnseite abschließt. In dem Verschlusskopf 16 ist eine Führungsbohrung 17 vorgesehen, in welcher die Kolbenstange 8 geführt ist.

An dem hinteren Ende der Kolbenstange 8 ist ein kreisscheibenförmiger Kolben 18 angeordnet, dessen Außendurchmesser dem Innendurchmesser des Hohlzylinders 6 entspricht. An die Rückseite des Kolbens 18 schließt ein Faltenbalg 19 an. Das hintere Ende des Faltenbalgs 19 ist an einem weiteren Verschlusskopf 20 befestigt, der den Hohlzylinder 6 an seiner hinteren Stirnseite abschließt. In den Verschlusskopf 20 mündet ein Druckluftanschluss ein, der von einer Lufttülle 21 gebildet ist.

Der Kolben 18 und die Kolbenstange 8 bestehen aus Aluminium oder nicht rostendem Stahl. Das Druckelement 15 besteht vorzugsweise aus Polyamid. Die Verschlussköpfe 16, 20 bestehen vorzugsweise aus Aluminium und sind am Hohlzylinder 6 angeschraubt, eingerastet oder eingegossen. Die Lufttülle 21 weist vorzugsweise ein Gewinde zum Einsetzen in eine entsprechende Öffnung in dem Verschlusskopf 20 und luftdichten Fixieren des Faltenbalgs 19 mit einer Rundmutter auf.

Der Faltenbalg 19 ist rotationssymmetrisch zur Symmetrieachse des Hohlzylinders 6 ausgebildet und besteht vorzugsweise aus einem hochelastischen, textilverstärkten Material, insbesondere aus Latex. Der Außendurchmesser des Faltenbalgs 19 ist geringfügig kleiner als der lnnendurchmesser des Hohlzylinders 6, so dass die Außenwand des Faltenbalgs 19 mit geringem Spiel entlang der Wand des Hohlzylinders 6 bewegbar ist.

Über den Druckluftanschluss wird Druckluft in den Faltenbalg 19 geleitet, wodurch sich der Faltenbalg 19 in Längsrichtung ausdehnt und eine vorgegebene Kraft auf den Kolben 18 ausgeübt wird. Dies bewirkt eine definierte Auslenkung der Kolbenstange 8 und damit eine bestimmte Quengelkraft.

Durch die Vorgabe des Druckes im Faltenbalg 19, der vorzugsweise innerhalb eines Bereiches zwischen 533,3 Pa und 333305 Pa (0,4 und 250 cm/Hg) variiert, ist eine präzise Einstellung der Auslenkung der Kolbenstange 8 und damit eine präzise und reproduzierbare Vorgabe der Quengelkraft gewährleistet. Die Maximalauslenkung der Kolbenstange 8 wird vorzugsweise mittels eines nicht dargestellten Endschalters erfasst.

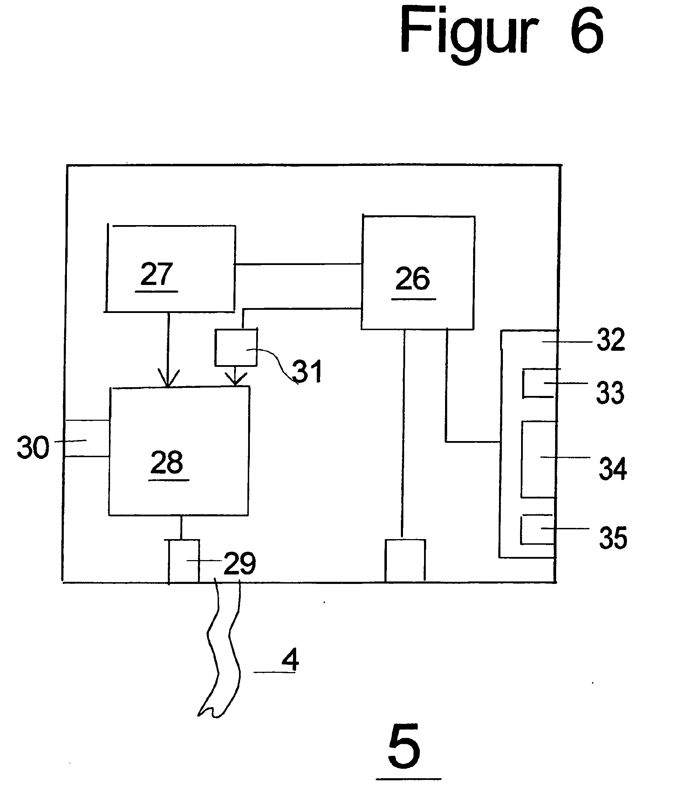

Mittels der in den Figuren 5 und 6 dargestellten Befestigungs- und Justiereinheit 11 kann die pneumatische Korrektureinheit 3 positionsverstellbar an jeder Quengel-Orthese 1 befestigt werden. Die Befestigungs- und Justiereinheit 11 weist zwei in Längsrichtung verlaufende, übereinander liegende Schienen 22, 23 auf. An der unteren Schiene 23 ist der Haltering 10 befestigt, der zur Fixie-rung der pneumatischen Korrektureinheit 3 die Mantelfläche des Hohlzylinders 6 umschließt. Die untere Schiene 23 wird an dem jeweiligen Orthesenteil 1a, befestigt, insbesondere angeschraubt.

Die Schienen 22, 23 weisen an ihren aneinander angrenzenden Grenzflächen ineinander greifende Zahnleisten 24, 25 auf. Wie aus Figur 5 ersichtlich weisen die Zahnleisten 24, 25 der Schienen 22, 23 jeweils in Längsrichtung der Schienen 22, 23 hintereinander angeordnete Zahnsegmente auf. Die Zahnsegmente weisen jeweils identische quadratische Querschnitte auf, die über die gesamte Breite der jeweiligen Schienen 22, 23 konstant sind. Zur Einstellung einer Sollposition der pneumatischen Korrektureinheit 3 an dem jeweiligen Orthesenteil 1a, 1b werden die Zahnleisten 24, 25 in einer entsprechenden Relativposition gegeneinander gesetzt und dann mit dem Haltering 10 gegeneinander fixiert.

Wie aus Figur 6 ersichtlich ist die erste Schiene 22 als Winkelschiene ausgebildet, während die zweite Schiene 23 als U-Schiene ausgebildet ist. In den Schienen 22, 23 sind die Zahnsegmente quer zur Schienenbreite bündig am jeweiligen Winkel verschraubt. Die Schiene 22 wird am Hohlzylinder befestigt. Zum Justieren in Längsrichtung wird die Schiene 22 mit den Zahnsegmenten von der Schiene 23 abgehoben, in der erforderlichen Länge verschoben und mit den Zahnsegmenten in die Zahnsegmente der Schiene 23 wieder eingesetzt. Der Haltering 10 fixiert die Schiene 22 mit dem Hohlzylinder 6 gegen die Schiene 23, die an der Quengel-Orthese 1 befestigt ist.

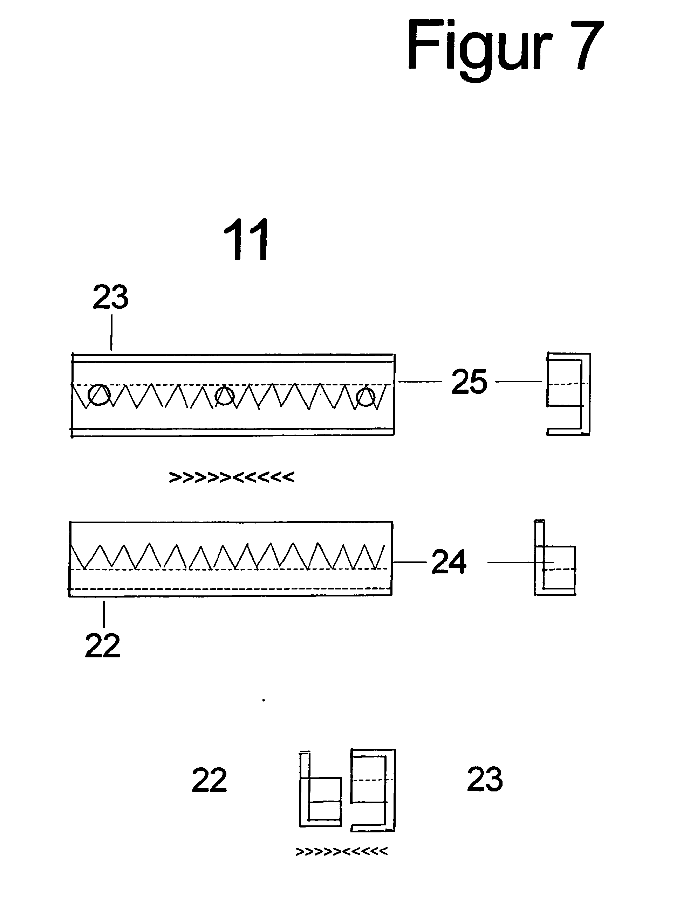

Figur 7 zeigt ein Blockschaltbild der Steuereinheit 5, mittels derer die pneumatische Korrektureinheit 3 gesteuert wird. Die Steuereinheit 5 ist in einem Gehäuse integriert und weist eine Rechnereinheit 26 auf, die von einem Mikrocontroller oder einem Mikroprozessor gebildet ist. Weiterhin weist die Steuereinheit 5 eine Pumpe 27, ein Luftreservoir 28 und einen Druckluftanschluss 29 auf. Die Pumpe 27 ist an die Rechnereinheit 26 angeschlossen und wird von dieser gesteuert.

Über die Pumpe 27 wird Druckluft durch das Luftreservoir 28 über den an den Druckluftanschluss 29 angeschlossenen Schlauch 4 in den Faltenbalg 19 der pneumatischen Korrektureinheit 3 eingespeist. Dabei ist das Volumen des Luftreservoirs 28 erheblich größer als das Volumen des Faltenbalgs 19. Vorzugsweise beträgt das Volumen des Luftreservoirs 28 etwa 250 ccm. Dadurch ist gewährleistet, dass über die Steuereinheit 5 der Luftdruck im Faltenbalg 19 schnell und präzise auf einen über die Rechnereinheit 26 vorgegebenen Sollwert nachgeführt werden kann, wodurch eine kontinuierliche Vorschubbewegung der Kolbenstange 8 erhalten wird.

Zur Reduzierung des Druckes im Luftreservoir 28 ist ein Ablassventil 30 vorgesehen, welches manuell oder über die Rechnereinheit 26 betätigbar ist.

Die Steuerung des Luftdruckes im Faltenbalg 19 der pneumatischen Korrektureinheit 3 und damit der erzeugten Quengelkraft erfolgt über die Rechnereinheit 26 durch eine geeignete Ansteuerung der Pumpe 27. Vorzugsweise erfolgt über die Rechnereinheit 26 eine Druckregelung, wobei der Luftdruck im Luftreservoir 28 mittels eines Druckwächters 31 überwacht wird.

Weiterhin ist an die Rechnereinheit 26 eine Ein-/Ausgabeeinheit 32 angeschlossen. Diese weist einen Schalter 33 zum Ein- und Ausschalten der Steuereinheit 5 auf. Zudem ist eine Bedienoberfläche 34 zur Eingabe von Parameterwerten in die Rechnereinheit 26 vorgesehen. Insbesondere sind dadurch die Sollwerte, auf welchen der Luftdruck mittels der Rechnereinheit 26 geregelt werden soll, und die Bereiche, innerhalb dessen der Luftdruck variieren kann, auf diese Weise einstellbar. Zudem sind über die Bedienoberfläche 34 die in der Rechnereinheit 26 generierten und gespeicherten Messwerte, insbesondere Druckwerte sowie Nutzungs- oder Gebrauchszeiten, ausgebbar, so dass diese während des gesamten Betriebs der Steuereinheit 5 kontrollierbar sind. Dadurch ist eine vollständige und umfassende Therapiekontrolle gewährleistet.

Die Bedienoberfläche 34 der Ein-/Ausgabeeinheit 32 weist insbesondere einen Anschluss für einen Drucker auf, mittels dessen die Messwerte über einen vorgegebenen Zeitraum aufgezeichnet werden können. Zudem weist die Bedienoberfläche 34 der Ein-/Ausgabeeinheit 32 eine Digitalanzeige zur Anzeige der aktuellen Messwerte auf. Schließlich weist die Bedienoberfläche 34 der Ein-/Ausgabeeinheit 32 ein Bedienteil zur Eingabe von Parameterwerten auf.

Die Steuereinheit 5 weist weiterhin einen elektrischen Anschluss auf, an welchen über eine Kabelverbindung der Endschalter in der pneumatischen Korrektureinheit 3 anschließbar ist. Dadurch kann in der Rechnereinheit 26 überwacht werden, ob die Endposition der Kolbenstange 8 erreicht ist. Das Erreichen der Endposition wird über die Ein-/Ausgabeeinheit 32 angezeigt. Vorzugsweise weist diese hierzu eine Leuchtdiode 35 auf.

Die Steuereinheit 5 weist schließlich eine nicht dargestellte Stromversorgung, insbesondere eine Batterie, auf. Besonders vorteilhaft ist dabei die Steuereinheit 5 in einem kompakten nicht dargestellten Gehäuse untergebracht, so dass diese mit der Quengel-Orthese 1 und der pneumatischen Korrektureinheit 3 eine tragbare und damit mobile Einheit bildet.

### Bezugszeichenliste

- (1): Quengel-Orthese
- (1a): Erstes Orthesenteil
- (1b): Zweites Orthesenteil
- (2): Gelenkverbindung
- (3): Pneumatische Korrektureinheit
- (4): Schlauch
- (5): Steuereinheit
- (6): Hohlzylinder
- (7): Erste Halterung
- (8): Kolbenstange
- (9): Zweite Halterung
- (10): Haltering
- (11): Befestigungs- und Justiereinheit
- (12): Befestigungsmittel
- (13): Erstes Haltearm
- (14): Zweiter Haltearm
- (15): Druckelement
- (16): Verschlusskopf
- (17): Führungsbohrung
- (18): Kolben
- (19): Faltenbalg
- (20): Verschlusskopf
- (21): Lufttülle
- (22): Schiene
- (23): Schiene
- (24): Zahnleiste
- (25): Zahnleiste
- (26): Rechnereinheit
- (27): Pumpe
- (28): Luftreservoir
- (29): Druckluftanschluss
- (30): Ablassventil
- (31): Druckwächter
- (32): Ein-/Ausgabeeinheit
- (33): Schalter
- (34): Bedienoberfläche
- (35): Leuchtdiode

## Patentansprüche

1. Quengel-Orthese zur Korrektur von Gelenk-Fehlstellungen mit zwei gelenkig miteinander verbundenen Orthesenteilen (1a, 1b) und mit einem Stellglied zur Erzeugung einer auf das Gelenk wirkenden Quengelkraft, **dadurch gekennzeichnet, dass** eine pneumatische Korrektureinheit (3) vorgesehen ist, welche als Grundkörper einen am ersten Orthesenteil (1a) befestigten Hohlzylinder (6) und als Stellglied eine im Hohlzylinder (6) verschiebbar gelagerte Kolbenstange (8) aufweist, dass das zweite Orthesenteil (1b) eine in Wirkverbindung mit dem Stellglied stehende Halterung (9) aufweist,so dass bei Betätigung des Stellglieds die Orthesenteile (1a, 1b) gegeneinander schwenkbar sind, und dass die Verschiebekraft und der Verschiebeweg der Kolbenstange (8) über den Luftdruck im Hohlzylinder (6) vorgebbar ist.

2. Quengel-Orthese nach Anspruch 1, **dadurch gekennzeichnet, dass** an der vorderen Stirnseite des Hohlzylinders (6) ein Verschlusskopf (16) mit einer Führungsbohrung (17) vorgesehen ist, in welcher die Kolbenstange (8) in Längsrichtung des Hohlzylinders (6) verschiebbar gelagert ist.

3. Quengel-Orthese nach einem der Ansprüche 1 oder 2, **dadurch gekennzeichnet, dass** an dem in den Hohlzylinder (6) ragenden hinteren Ende der Kolbenstange (8) ein Kolben (18) angeordnet ist, dessen Durchmesser dem Innendurchmesser des Hohlzylinders (6) entspricht und an dessen Rückseite ein mit Druckluft beaufschlagbarer Faltenbalg (19) anschließt.

4. Quengel-Orthese nach Anspruch 3, **dadurch gekennzeichnet, dass** das hintere Ende des Faltenbalgs (19) an einen Verschlusskopf (20) an der hinteren Stirnseite des Hohlzylinders (6) anschließt, wobei in dem Verschlusskopf (20) ein Druckluftanschluss zur Einspeisung von Druckluft in den Faltenbalg (19) angeordnet ist.

5. Quengel-Orthese nach Anspruch 4, **dadurch gekennzeichnet, dass** der Druckluftanschluss von einer Lufttülle (21) gebildet ist.

6. Quengel-Orthese nach einem der Ansprüche 3 bis 5, **dadurch** gekennzeichnen, dass der Faltenbalg (19) aus einem elastischen, textilverstärkten Material, insbesondere Latex, besteht.

7. Quengel-Orthese nach einem der Ansprüche 3 bis 6, **dadurch gekennzeichnet, dass** der Faltenbalg (19) symmetrisch zur Längsachse des Hohlzylinders (6) ausgebildet ist, wobei dessen Außendurchmesser an den Innendurchmesser des Hohlzylinders (6) angepasst ist.

8. Quengel-Orthese nach einem der Ansprüche 1 bis 7, **dadurch gekennzeichnet, dass** die das Stellglied bildende Kolbenstange (8) an ihrem Vorderende ein kugelförmiges Druckelement (15) aufweist, welches an einem ein Widerlager bildenden Segment der zugeordneten Halterung (9) anliegt.

9. Quengel-Orthese nach Anspruch 8, **dadurch gekennzeichnet, dass** der den Grundkörper bildende Hohlzylinder (6) zur Fixierung am ersten Orthesenteil (1a) an seiner Mantelfläche von einem Haltering (10) umschlossen ist.

10. Quengel-Orthese nach Anspruch 9, **dadurch gekennzeichnet, dass** der Haltering (10) Bestandteil eines von dem ersten Orthesenteils (1a) abstehenden, die Halterung (7) bildenden Haltearms (13) ist.

11. Quengel-Orthese nach Anspruch 10, **dadurch gekennzeichnet, dass** der Haltering (10) Bestandteil einer die Halterung (7) bildenden Befestigungs- und Justiereinheit (11) ist, mittels derer der Hohlzylinder (6) positionsverstellbar am ersten Orthesenteil (1a) befestigbar ist.

12. Quengel-Orthese nach Anspruch 11, **dadurch gekennzeichnet, dass** die Befestigungs- und Justiereinheit (11) zwei in Längsrichtung des Hohlzylinders (6) verlaufende, ineinander liegende Schienen (22, 23) aufweist, wobei die Schienen (22, 23) an ihren ineinander greifenden Winkelflächen formschlüssig ineinander greifende Zahnleisten (24, 25) aufweisen.

13. Quengel-Orthese nach Anspruch 12, **dadurch gekennzeichnet, dass** die Schienen (22, 23) in einstellbaren Relativpositionen mittels des Halterings (10) fixierbar sind.

14. Quengel-Orthese nach einem der Ansprüche 12 oder 13, **dadurch gekennzeichnet, dass** die obere Schiene (22) am Hohlzylinder (6) befestigt ist, und dass die untere Schiene (23) mittels Befestigungsmitteln (12) an dem ersten Orthesenteil (1a) befestigt ist.

15. Quengel-Orthese nach einem der Ansprüche 1 bis 14, **dadurch gekennzeichnet, dass** die Steuereinheit (5) ein Luftreservoir (28), eine Pumpe (27) und einen Druckluftanschluss (29) aufweist, wobei mittels der Pumpe (27) Druckluft durch das Luftreservoir (28) über den Druckluftanschluss (29) der Korrektureinheit zuführbar ist.

16. Quengel-Orthese nach Anspruch 15, **dadurch gekennzeichnet, dass** die Druckluftanschlüsse (29) an der Steuereinheit (5) und am Hohlzylinder (6) über einen Schlauch (4) verbunden sind.

17. Quengel-Orthese nach einem der Ansprüche 15 oder 16, **dadurch gekennzeichnet, dass** das Volumen des Luftreservoirs (28) erheblich größer als das Volumen des Hohlzylinders (6) ist.

18. Quenge1-Orthese nach einem der Ansprüche 15 bis 17, **dadurch gekennzeichnet, dass** die Steuereinheit (5) eine Rechnereinheit (26) zur Vorgabe des Druckes in der Korrektureinheit aufweist.

19. Quengel-Orthese nach Anspruch 18, **dadurch gekennzeichnet, dass** mittels der Rechnereinheit (26) die Pumpe (27) angesteuert wird.

20. Quengel-Orthese nach einem der Ansprüche 18 oder 19, **dadurch gekennzeichnet, dass** mittels der Rechnereinheit (26) eine Druckregelung erfolgt.

21. Quengel-Orthese nach einem der Ansprüche 18 bis 20, **dadurch gekennzeichnet, dass** der Rechnereinheit (26) eine Ein-/Ausgabeeinheit (32) zugeordnet ist.

22. Quengel-Orthese nach Anspruch 21, **dadurch gekennzeichnet, dass** über die Ein-/Ausgabeeinheit (32) Sollwerte für die Einstellung des Druckes vorgebbar sind.

23. Quengel-Orthese nach Anspruch 22, **dadurch gekennzeichnet, dass** über die Ein-/Ausgabeeinheit (32) in der Rechnereinheit (26) generierte und abgespeicherte Nutzungsdaten, insbesondere das Datum und die Betriebsdauer, ausgebbar sind.

24. Quengel-Orthese nach einem der Ansprüche 22 oder 23, **dadurch gekennzeichnet, dass** an die Rechnereinheit (26) ein Druckwächter (31) zur Kontrolle des eingestellten Druckes im Luftreservoir (28) angeschlossen ist, wobei die vom Druckwächter (31) generierten Messwerte über die Ein-/Ausgabeeinheit (32) ausgebbar sind.

25. Quengel-Orthese nach einem der Ansprüche 22 bis 24, **dadurch gekennzeichnet, dass** an die Rechnereinheit (26) ein in der Korrektureinheit angeordneter Endschalter zur Erfassung der Endposition der Kolbenstange (8) angeschlossen ist, und dass das Erreichen der Endposition über die Ein-/Ausgabeeinheit (32) anzeigbar ist.

26. Quengel-Orthese nach Anspruch 25, **dadurch gekennzeichnet, dass** die Ein-/Ausgabeeinheit (32) zur Anzeige der Endposition der Kolbenstange (8) eine Leuchtdiode (35) aufweist.

27. Quengel-Orthese nach einem der Ansprüche 1 bis 26, **dadurch gekennzeichnet, dass** die Steuereinheit (5) eine Stromversorgungseinheit aufweist.

28. Quengel-Orthese nach einem der Ansprüche 1 bis 27, **dadurch gekennzeichnet, dass** diese mit der Korrektureinheit und der Steuereinheit (5) eine tragbare Einheit bildet.

## Claims

1. Quengel orthesis for correction of faulty joint settings, comprising two pivotably interconnected orthesis parts (1 a, 1 b) and a setting element for producing a Quengel force acting on a joint, **characterised in that** a pneumatic correction unit (3) is provided, which has as base body a hollow cylinder (6) fastened to the first orthesis part (1a) and as setting element a piston rod (8) displaceably mounted in the hollow cylinder (6), that the second orthesis part (1 b) has a retainer (9) disposed in operative connection with the setting element so that on actuation of the setting element the orthesis parts (1 a, 1 b) are pivotable relative to one another, and that the displacing force and the displacement travel of the piston rod (8) is predeterminable by way of the air pressure in the hollow cylinder (6).

2. Quengel orthesis according to claim 1, **characterised in that** a closure head (16) with a guide bore (17) in which the piston rod (8) is mounted to be displaceable in longitudinal direction of the hollow cylinder (6) is provided at the front side of the hollow cylinder (6).

3. Quengel orthesis according to one of claims 1 and 2, **characterised in that** a piston (18), the diameter of which corresponds with the inner diameter of the hollow cylinder (6) and connected with the rear side of which is a bellows (19) loadable with compressed air, is arranged at the rear end, which projects into the hollow cylinder (6) of the piston rod (8).

4. Quengel orthesis according to claim 3, **characterised in that** the rear end of the bellows (19) is connected with a closure head (20) at the rear end face of the hollow cylinder (6), wherein a compressed air connection for supply of compressed air to the bellows (19) is arranged in the closure head (20).

5. Quengel orthesis according to claim 4, **characterised in that** the compressed air connection is formed by an air nozzle (21).

6. Quengel orthesis according to one of claims 3 to 5, **characterised in that** the bellows (19) consists of a resilient textile-reinforced material, particularly latex.

7. Quengel orthesis according to one of claims 3 to 6, **characterised in that** the bellows (19) is formed symmetrically with respect to the longitudinal axis of the hollow cylinder (6), wherein the outer diameter thereof is matched to the inner diameter of the hollow cylinder (6).

8. Quengel orthesis according to one of claims 1 to 7, **characterised in that** the piston rod (8) forming the setting element has at its front end a spherical pressure element (15) bearing against a segment, which forms a counter-bearing, of the associated retainer (9).

9. Quengel orthesis according to claim 8, **characterised in that** the hollow cylinder (6) forming the base body is, for fixing to the first orthesis part (1a), surrounded at its circumferential surface by a retaining ring (10).

10. Quengel orthesis according to claim 9, **characterised in that** the retaining ring (10) is a component of a retaining arm (13) which protrudes from the first orthesis part (1 a) and forms the retainer (7).

11. Quengel orthesis according to claim 10, **characterised in that** the retaining ring (10) is a component of a fastening and adjusting unit (11) which forms the retainer (7) and by means of which the hollow cylinder (6) is fastenable to the first orthesis part (1 a) to be adjustable in position.

12. Quengel orthesis according to claim 11, **characterised in that** the fastening and adjusting unit (11) comprises two rails (22, 23) which extend in longitudinal direction of the hollow cylinder (6) and lie one in the other, wherein the rails (22, 23) have mechanically positively interengaging toothed strips (24, 25) at their interengaging angle surfaces.

13. Quengel orthesis according to claim 12, **characterised in that** the rails (22, 23) are fixable in settable relative positions by means of the retaining ring (10).

14. Quengel orthesis according to one of claims 12 and 13, **characterised in that** the upper rail (22) is fastened to the hollow cylinder and that the lower rail (23) is fastened to the first orthesis part (1 a) by way of fastening means (12).

15. Quengel orthesis according to one of claims 1 to 14, **characterised in that** the control unit (5) comprises an air reservoir (28), a pump (27) and a compressed air connection (29), wherein compressed air can be fed by means of the pump (27) through the air reservoir (28) via the compressed air connection (29) to the correction unit.

16. Quengel orthesis according to claim 15, **characterised in that** the compressed air connections (29) at the control unit (5) and the hollow cylinder (6) are connected by way of a hose (4).

17. Quengel orthesis according to one of claims 15 and 16, **characterised in that** the volume of the air reservoir (28) is substantially greater than the volume of the hollow cylinder (6).

18. Quengel orthesis according to one of claims 15 to 17, **characterised in that** the control unit (5) comprises a computer unit (26) for presetting the pressure in the correction unit.

19. Quengel orthesis according to claim 18, **characterised in that** the pump (27) is controlled in drive by means of the computer unit (26).

20. Quengel orthesis according to one of claims 18 and 19, **characterised in that** a pressure regulation is carried out by means of the computer unit (26).

21. Quengel orthesis according to one of claims 18 to 20, **characterised in that** an input/output unit (32) is associated with the computer unit (26).

22. Quengel orthesis according to claim 21, **characterised in that** target values for setting the pressure are presettable by way of the input/output unit (32).

23. Quengel orthesis according to claim 22, **characterised in that** use data, particularly the date and operating duration, generated and stored in the computer unit (26) can be output by way of the input/output unit (32).

24. Quengel orthesis according to one of claims 22 and 23, **characterised in that** a pressure monitor (31) for checking the set pressure in the air reservoir (28) is connected with the computer unit (26), wherein the measurement values generated by the pressure monitor (31) can be output by way of the input/output unit (32).

25. Quengel orthesis according to one of claims 22 to 24, **characterised in that** a limit switch, which is arranged in the correction unit, for detection of the end position of the piston rod (8) is connected with the computer unit (26) and that attainment of the end position can be indicated by way of the input/output unit (32).

26. Quengel orthesis according to claim 25, **characterised in that** the input/output unit (32) has a light-emitting diode (35) for indicating the end position of the piston rod (8).

27. Quengel orthesis according to one of claims 1 to 26, **characterised in that** the control unit (5) has a power supply unit.

28. Quengel orthesis according to one of claims 1 to 27, **characterised in that** this forms, together with the correction unit and the control unit (5), a portable unit.

## Revendications

1. Orthèse de type Quengel pour la correction de postures articulaires défectueuses, comprenant deux parties (1a, 1b) reliées l'une à l'autre de manière articulée, et un organe de manoeuvre pour engendrer une force correctrice agissant sur l'articulation, **caractérisée par** la présence d'une unité correctrice pneumatique (3) comportant, en tant que corps de base, un cylindre creux (6) relié à la première partie (1a) de l'orthèse et, en tant qu'organe de manoeuvre, une tige (8) de piston logée à coulissement dans ledit cylindre creux (6) ; par le fait que la seconde partie (1b) de l'orthèse présente une pièce de retenue (9) en liaison interactive avec l'organe de manoeuvre, de façon telle que les parties (la, 1b) de l'orthèse puissent pivoter l'une par rapport à l'autre lors d'un actionnement dudit organe de manoeuvre ; et par le fait que la force de coulissement et le trajet de coulissement de la tige (8) de piston peuvent être préétablis par la pression de l'air régnant dans le cylindre creux (6).

2. Orthèse de type Quengel selon la revendication 1, **caractérisée par** la présence, à la face extrême antérieure du cylindre creux (6), d'une tête obturatrice (16) munie d'un alésage de guidage (17) dans lequel la tige (8) de piston est montée à coulissement dans la direction longitudinale dudit cylindre creux (6).

3. Orthèse de type Quengel selon l'une des revendications 1 ou 2, **caractérisée par le fait qu'**un piston (18) est disposé à l'extrémité postérieure de la tige (8) qui s'engage dans le cylindre creux (6), le diamètre dudit piston correspondant au diamètre intérieur dudit cylindre creux (6) et un soufflet (19), pouvant être sollicité par de l'air comprimé, se rattachant à la face postérieure dudit piston.

4. Orthèse de type Quengel selon la revendication 3, **caractérisée par le fait que** l'extrémité postérieure du soufflet (19) se rattache à une tête obturatrice (20), à la face extrême postérieure du cylindre creux (6), un raccord à air comprimé étant disposé dans ladite tête obturatrice (20), en vue d'introduire de l'air comprimé dans ledit soufflet (19).

5. Orthèse de type Quengel selon la revendication 4, **caractérisée par le fait que** le raccord à air comprimé est matérialisé par un embout (21) à air.

6. Orthèse de type Quengel selon l'une des revendications 3 à 5, **caractérisée par le fait que** le soufflet (19) est constitué d'un matériau élastique, notamment du latex, muni d'un renfort textile.

7. Orthèse de type Quengel selon l'une des revendications 3 à 6, **caractérisée par le fait que** le soufflet (19) est de réalisation symétrique par rapport à l'axe longitudinal du cylindre creux (6), sachant que son diamètre extérieur est adapté au diamètre intérieur dudit cylindre creux (6).

8. Orthèse de type Quengel selon l'une des revendications 1 à 7, **caractérisée par le fait que** la tige (8) de piston matérialisant l'organe de manoeuvre comporte, à son extrémité antérieure, un élément sphérique de pression (15) appliqué contre un segment de la pièce de retenue (9) associée, qui matérialise une contre-butée.

9. Orthèse de type Quengel selon la revendication 8, **caractérisée par le fait que** le cylindre creux (6) matérialisant le corps de base est entouré par une bague d'arrêt (10), sur la surface de son enveloppe, en vue de la consignation à demeure sur la première partie (1a) de ladite orthèse.

10. Orthèse de type Quengel selon la revendication 9, **caractérisée par le fait que** la bague d'arrêt (10) fait partie intégrante d'un bras d'arrêt (13) matérialisant la pièce de retenue (7) et faisant saillie au-delà de la première partie (1a) de ladite orthèse.

11. Orthèse de type Quengel selon la revendication 10, **caractérisée par le fait que** la bague d'arrêt (10) fait partie intégrante d'une unité (11) de fixation et d'ajustement matérialisant la pièce de retenue (7) et au moyen de laquelle le cylindre creux (6) peut être fixé à la première partie (1a) de ladite orthèse, avec faculté de positionnement réglable.

12. Orthèse de type Quengel selon la revendication 11, **caractérisée par le fait que** l'unité (11) de fixation et d'ajustement comporte deux glissières (22, 23) imbriquées l'une dans l'autre et s'étendant dans la direction longitudinale du cylindre creux (6), lesdites glissières (22, 23) présentant, au niveau de leurs surfaces coudées s'interpénétrant, des barrettes crantées (24, 25) à interpénétration par concordance de formes.

13. Orthèse de type Quengel selon la revendication 12, **caractérisée par le fait que** les glissières (22, 23) peuvent être bloquées dans des positions relatives réglables, au moyen de la bague d'arrêt (10).

14. Orthèse de type Quengel selon l'une des revendications 12 ou 13,
**caractérisée par le fait que** la glissière supérieure (22) est fixée au cylindre creux (6) ; et **par le fait que** la glissière inférieure (23) est fixée à la première partie (1a) de ladite orthèse grâce à des moyens de fixation (12).

15. Orthèse de type Quengel selon l'une des revendications 1 à 14, **caractérisée par le fait que** l'unité de commande (5) comprend un réservoir d'air (28), une pompe (27) et un raccord (29) à air comprimé, sachant que de l'air comprimé peut être délivré à l'unité correctrice au moyen de ladite pompe (27), par l'intermédiaire dudit raccord (29) à air comprimé, en traversant ledit réservoir d'air (28).

16. Orthèse de type Quengel selon la revendication 15, **caractérisée par le fait que** les raccords (29) à air comprimé sont reliés à l'unité de commande (5) et au cylindre creux (6) par l'intermédiaire d'un tuyau souple (4).

17. Orthèse de type Quengel selon l'une des revendications 15 ou 16, **caractérisée par le fait que** le volume du réservoir d'air (28) et notablement plus grand que le volume du cylindre creux (6).

18. Orthèse de type Quengel selon l'une des revendications 15 à 17, **caractérisée par le fait que** l'unité de commande (5) présente une unité de calcul (26), en vue de préétablir la pression régnant dans l'unité correctrice.

19. Orthèse de type Quengel selon la revendication 18, **caractérisée par le fait que** la pompe (27) est activée au moyen de l'unité de calcul (26).

20. Orthèse de type Quengel selon l'une des revendications 18 ou 19, **caractérisée par le fait qu'**une régulation de pression a lieu au moyen de l'unité de calcul (26).

21. Orthèse de type Quengel selon l'une des revendications 18 à 20, **caractérisée par le fait qu'**une unité (32) d'entrée/sortie est affectée à l'unité de calcul (26).

22. Orthèse de type Quengel selon la revendication 21, **caractérisée par le fait que** des valeurs de consigne, destinées au réglage de la pression, peuvent être préétablies par l'intermédiaire de l'unité (32) d'entrée/sortie.

23. Orthèse de type Quengel selon la revendication 22, **caractérisée par le fait que** des données d'utilisation engendrées et mémorisées dans l'unité de calcul (26), en particulier la date et la durée de service, peuvent être délivrées par l'intermédiaire de l'unité (32) d'entrée/sortie.

24. Orthèse de type Quengel selon l'une des revendications 22 ou 23, **caractérisée par le fait qu'**un moniteur de pression (31) est raccordé à l'unité de calcul (26) en vue de contrôler la pression réglée régnant dans le réservoir d'air (28), sachant que les valeurs mesurées, engendrées par ledit moniteur de pression (31), peuvent être délivrées par l'intermédiaire de l'unité (32) d'entrée/sortie.

25. Orthèse de type Quengel selon l'une des revendications 22 à 24,
**caractérisée par le fait qu'**un interrupteur de fin de course, intégré dans l'unité correctrice, est connecté à l'unité de calcul (26) en vue de détecter la position extrême de la tige (8) de piston ; et **par le fait que** l'arrivée à ladite position extrême peut être affichée par l'intermédiaire de l'unité (32) d'entrée/sortie.

26. Orthèse de type Quengel selon la revendication 25, **caractérisée par le fait que** l'unité (32) d'entrée/sortie présente une diode électroluminescente (35) en vue d'afficher la position extrême de la tige (8) de piston.

27. Orthèse de type Quengel selon l'une des revendications 1 à 26, **caractérisée par le fait que** l'unité de commande (5) comporte une unité d'alimentation en courant.

28. Orthèse de type Quengel selon l'une des revendications 1 à 27, **caractérisée par le fait que** ladite orthèse forme un ensemble unitaire portable en association avec l'unité correctrice et avec l'unité de commande (5).
